(19) Europäisches Patentamt
European Patent Office
Office européen des brevets

(11) Veröffentlichungsnummer: **0 046 264 B1**

# (12) EUROPÄISCHE PATENTSCHRIFT

(45) Veröffentlichungstag der Patentschrift: **20.06.84**

(51) Int. Cl.³: **C 07 C 103/78**, C 07 C 103/84, C 07 D 263/42, C 07 C 102/04

(21) Anmeldenummer: **81106244.7**

(22) Anmeldetag: **11.08.81**

## (54) Verfahren zur Herstellung von N-(2-(p-Hydroxyphenyl)-ethyl)-p-chlorbenzamid und Zwischenprodukte zur Herstellung dieser Verbindung.

(30) Priorität: **14.08.80 US 177906**

(43) Veröffentlichungstag der Anmeldung: **24.02.82 Patentblatt 82/8**

(45) Bekanntmachung des Hinweises auf die Patenterteilung: **20.06.84 Patentblatt 84/25**

(84) Benannte Vertragsstaaten: **AT BE CH DE FR GB IT LI LU NL SE**

(56) Entgegenhaltungen:
**DE - A - 2 659 543**
**DE - B - 2 149 070**

**THE JOURNAL OF ORGANIC CHEMISTRY, Band 41, Nr. 4, 20. Februar 1976 Y.S. RAO "A New Stereospecific Synthesis of the E Isomeres of 2-Phenyl-4-Arylemthylene-2-oxazoline-5-ones" Seiten 722 bis 725**
**W. KUNZ et al. "Reaktionen der organischen Chemie" 5. Auflage 1976, DR. ALFRED HUTHIG VERLAG, Heidelberg Seiten 180 bis 181**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Harris, Nicholas, Howard Street, Norwich New York 13815 (US)**

EP 0 046 264 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft die Herstellung von Bezafibrat – 2-{p-[2-(p-Chlorbenzamido)ethyl]phenoxy}-2-methylpropionsäure –, einem stark hypolipidämischen Wirkstoff, der in der US-Patentschrift Nr. 3781328 beschrieben ist. Gemäss dem dort dargestellten Syntheseweg wird Bezafibrat in der letzten Synthesestufe aus der Verbindung N-[2-(p-Hydroxyphenyl)ethyl]-p-chlorbenzamid durch Umsetzen mit 2-Brom-2-methylpropionsäure und anschliessende Hydrolyse gewonnen. Die Herstellung von N-[2-(p-Hydroxyphenyl)ethyl]-p-chlorbenzamid, dem unmittelbaren Vorprodukt des Bezafibrats, geschieht in der Regel über das sehr teuere Tyramin.

Es war Aufgabe der vorliegenden Erfindung, einen weiteren Syntheseweg für Bezafibrat zu finden, der die Verwendung von Tyramin vermeidet. Diese Aufgabe wurde dadurch gelöst, dass man von N-(p-Chlorbenzoyl)glycin ausgeht und dieses durch an sich bekannte Verfahrensschritte in das N-[2-(p-Hydrxyphenyl)ethyl]-p-chlorbenzamid überführt. Der Syntheseweg ist im folgenden Schema dargestellt.

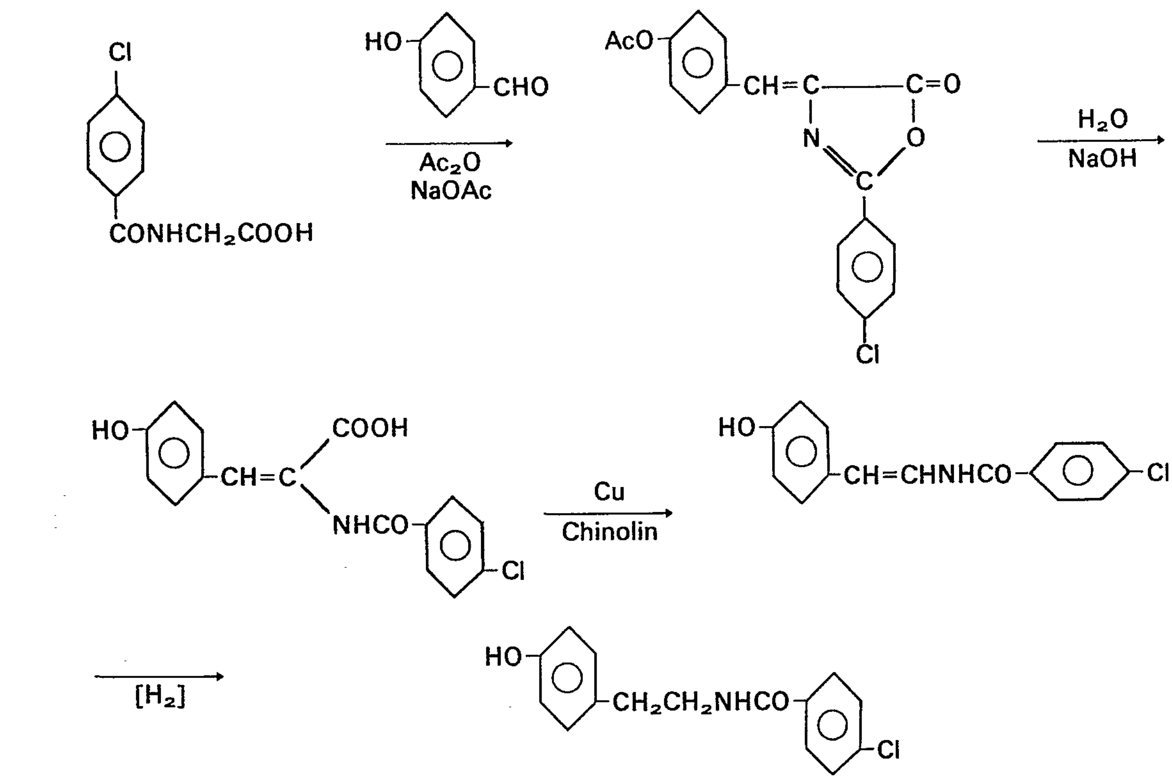


und umfasst folgende Stufen:

a) Umsetzung von N-(p-Chlorbenzoyl)glycin mit p-Hydroxybenzaldehyd unter den Bedingungen einer Aldolkondensation zu einem 4-(p-Acyloxybenzyliden)-2-(p-chlorphenyl)-5-oxazolon,

b) basische Verseifung des 4-(p-Acyloxybenzyliden)-2-(p-chlorphenyl)-5-oxazolons zu 2-(p-Chlorbenzamido)-3-(p-hydroxyphenyl)acrylsäure,

c) Decarboxylierung von 2-(p-Chlorbenzamido)-3-(p-hydroxyphenyl)acrylsäure zu 1-(p-Chlorbenzamid)-2-(p-hydroxyphenyl)ethylen, und

d) Hydrierung von 1-(p-Chlorbenzamido)-2-(p-hydroxyphenyl)ethylen zu N-[2-(p-Hydroxyphenyl)ethyl]-p-chlorbenzamid.

Unter einer Aldolkondensation gemäss Stufe a wird hier und im folgenden die Umsetzung von Aldehyden mit Ketonen als C-H-aziden Verbindungen unter basischen Bedingungen verstanden. Die spezielle Umsetzung gemäss Stufe a zu einem Oxazolonsystem wird in der Literatur auch als Erlenmeyer-Reaktion bezeichnet und läuft in Anwesenheit einer Base (vorzugsweise Natriumacetat) und einem wasserentziehenden Mittel (vorzugsweise Acetanhydrid) ab.

Die Verseifung gemäss Stufe b erfolgt unter basischen Bedingungen, z.B. in Anwesenheit von Alkalihydroxiden, vorzugsweise Natriumhydroxid.

Für die Decarboxylierung gemäss Stufe c kommt eine Reihe von literaturbekannten Methoden in Frage. Bevorzugt ist hier die Umsetzung mit Kupferpulver/Chinolin bei höheren Temperaturen.

Für die Hydrierung gemäss Stufe d kommen alle Methoden in Frage, die geeignet sind, vinylische Doppelbindungen neben einem in Konjugation befindlichen aromatischen Kern selektiv anzugreifen. Eine solche Methode ist beispielsweise die katalytische Hydrierung an Edelmetallen oder deren Oxiden. Bevorzugt ist hier die Hydrierung an Platinoxid.

Die als Zwischenstufen bei der Synthese von N-[2-(p-Hydroxyphenyl)ethyl]-p-chlorbenzamid auftretenden Verbindungen

4-(p-Acyloxybenzyliden)-2-(p-chlorphenyl)-5-oxazolon,
2-(p-Chlorbenzamido)-3-(p-hydroxyphenyl)-acrylsäure, und
1-(p-Chlorbenzamid)-2-(p-hydroxyphenyl)-ethylen
sowie deren Salze, Ester und Amide sind neu und stellen wertvolle Zwischenverbindungen zur Herstellung von pharmakologisch wirksamen Substanzen (wie z.B. Bezafibrat) dar.

Das Verfahren wird im folgenden anhand eines Beispiels erläutert. Es soll den Gegenstand der Erfindung dem Fachmann verdeutlichen, ihn aber in keiner Weise einschränken.

*Beispiel*

*Stufe A: 4-(p-Acetoxybenzyliden)-2-(p-chlorphenyl)-5-oxazolon*

Eine Mischung von 60 g N-(p-Chlorbenzoyl)-glycin, 34,5 g p-Hydroxybenzaldehyd und 23 g Natriumacetat *(pro analysi)* wurde in 170 ml Acetanhydrid 1 h am Rückfluss gekocht. Die Reaktionsmischung wurde etwas abgekühlt und mit 400 ml Ethanol versetzt. Beim vollständigen Abkühlen fiel ein fester Niederschlag aus. Dieser wurde abfiltriert und mit kaltem Ethanol und kaltem Wasser gewaschen. Eine analytische Probe wurde aus $CCl_4$/EtOH/AcOH umkristallisiert. Fp. 185-186°C.

*Stufe B: 2-(p-Chlorbenzamido)-3-(p-hydroxyphenyl)acrylsäure*

Eine Mischung aus 48 g des substituierten 5-Oxazolons (aus Stufe A), 290 ml Aceton, 120 ml Wasser und 75 ml einer 28%igen wässerigen Natriumhydroxidlösung wurde 1 h unter Rückfluss gekocht. Nach dem Abkühlen wurde das ausgefallene feste Produkt gesammelt und mit Wasser gewaschen. Eine analytische Probe wurde aus Wasser/Essigsäure umkristallisiert. Fp. 239-240°C (unter Zers.).

*Stufe C: 1-(p-Chlorbenzamido)-2-(p-hydroxyphenyl)ethylen*

Eine Mischung aus 30 g der substituierten Acrylsäure (aus Stufe B), 3,2 g Kupferpulver, 250 ml Chinolin und 0,1 g t-Butylcatechol wurde unter Stickstoffatmosphäre 30 min auf 230-238°C erhitzt. Die abgekühlte Reaktionsmischung wurde filtriert und das Filtrat auf verdünnte Salzsäure im Überschuss gegeben. Es fiel ein fester Niederschlag aus, der gesammelt und mit Wasser gewaschen wurde. Eine analytische Probe wurde aus 80%igem wässerigen Ethanol umkristallisiert. Fp. 243-244°C.

*Stufe D: N-[2-(p-Hydroxyphenyl)ethyl]-p-chlorbenzamid*

5 g 1-(p-Chlorbenzamido)-2-(p-hydroxyphenyl)ethylen (aus Stufe C) wurden in 150 ml einer 1:1-Mischung von Methanol und Ethanol in Anwesenheit von 0,1 g Platinoxid in einem Parr-Schüttler bei 3 Atmosphären Wasserstoffdruck und Raumtemperatur in 15 min reduziert. Filtrieren und Eindampfen erbrachten 4,5 g des Produktes. Eine analytische Probe wurde aus verdünntem wässerigem Ethanol umkristallisiert. Fp. 170-172°C.

## Patentansprüche

1. Verfahren zur Herstellung von N-[2-(p-Hydroxyphenyl)ethyl]-p-chlorbenzamid dadurch gekennzeichnet, dass es folgende Stufen umfasst:

a) Umsetzung von N-(p-Chlorbenzoyl)glycin mit p-Hydroxybenzaldehyd unter den Bedingungen einer Aldolkondensation zu einem 4-(p-Acyloxybenzyliden)-2-(p-chlorphenyl)-5-oxazolon,

b) basische Verseifung des 4-(p-Acyloxybenzyliden)-2-(p-chlorphenyl)-5-oxazolons zu 2-(p-Chlorbenzamido)-3-(p-hydroxyphenyl)acrylsäure,

c) Decarboxylierung von 2-(p-Chlorbenzamid)-3-(p-hydroxyphenyl)acrylsäure zu 1-(p-Chlorbenzamido)-2-(p-hydroxyphenyl)ethylen, und

d) Hydrierung von 1-(p-Chlorbenzamido)-2-(p-hydroxyphenyl)ethylen zu N-[2-(p-Hydroxyphenyl)ethyl]-p-chlorbenzamid.

2. Verfahren zur Herstellung von N-[2-(p-Hydroxyphenyl)ethyl]-p-chlorbenzamid dadurch gekennzeichnet, dass es folgende Stufen umfasst:

a) Umsetzung von N-(p-Chlorbenzoyl)glycin mit p-Hydroxybenzaldehyd in Gegenwart von Natriumacetat und Acetanhydrid zu 4-(p-Acetoxybenzyliden)-2-(p-chlorphenyl)-5-oxazolon,

b) Verseifung von 4-(p-Acetoxybenzyliden)-2-(p-chlorphenyl)-5-oxazolon zu 2-(p-Chlorbenzamido)-3-(p-hydroxyphenyl)acrylsäure durch Behandeln mit Natriumhydroxid,

c) Decarboxylierung von 2-(p-Chlorbenzamido)-3-(p-hydroxyphenyl)acrylsäure zu 1-(p-Chlorbenzamido)-2-(p-hydroxyphenyl)ethylen in Anwesenheit von Kupfer und Chinolin, und

d) katalytische Hydrierung von 1-(p-Chlorbenzamido)-2-(p-hydroxyphenyl)ethylen zu N-[2-(p-Hydroxyphenyl)ethyl]-p-chlorbenzamid.

3. 4-(p-Acetoxybenzyliden)-2-(p-chlorphenyl)-5-oxazolon.

4. 2-(p-Chlorbenzamido)-3-(p-hydroxyphenyl)acrylsäure, deren Salze, Ester und Amide.

5. 1-(p-Chlorbenzamido)-2-(p-hydroxyphenyl)ethylen, dessen Salze und Ester.

## Claims

1. Process for the preparation of N-[2-(p-hydroxyphenyl)-ethyl]-p-chlorobenzamide, characterised in that it includes the following steps—

(*a*) reaction of N-(p-chlorobenzoyl)-glycine with p-hydroxybenzaldehyde under the conditions of an aldol condensation to give a 4-(p-acyloxybenzylidene)-2-(p-chlorophenyl)-5-oxazolone;

(*b*) basic saponification of the 4-(p-acyloxy-

benzylidene)-2-(p-chlorophenyl)-5-oxazolone to give 2-(p-chlorobenzamido)-3-(p-hydroxyphenyl)-acrylic acid;

(*c*) decarboxylation of 2-(p-chlorobenzamido)-3-(p-hydroxyphenyl)-acrylic acid to give 1-(p-chlorobenzamido)-2-(p-hydroxyphenyl)-ethylene, and

(*d*) hydrogenation of 1-(p-chlorobenzamido)-2-(p-hydroxyphenyl)-ethylene to give N-[2-(p-hydroxyphenyl)-ethyl]-p-chlorobenzamide.

2. Process for the preparation of N-[2-(p-hydroxyphenyl)-ethyl]-p-chlorobenzamide, characterised in that it includes the following steps—

(*a*) reaction of N-(p-chlorobenzoyl)-glycine with p-hydroxybenzaldehyde in the presence of sodium acetate and acetic anhydride to give 4-(p-acetoxybenzylidene)-2-(p-chlorophenyl)-5-oxazolone;

(*b*) saponification of 4-(p-acetoxybenzylidene)-2-(p-chlorophenyl)-5-oxazolone to give 2-(p-chlorobenzamido)-3-(p-hydroxyphenyl)-acrylic acid by treatment with sodium hydroxde;

(*c*) decarboxylation of 2-(p-chlorobenzamido)-3-(p-hydroxyphenyl)-acrylic acid to give 1-(p-chlorobenzamido)-2-(p-hydroxyphenyl)-ethylene in the presence of copper and quinoline, and

(*d*) catalytic hydrogenation of 1-(p-chlorobenzamido)-2-(p-hydroxyphenyl)-ethylene to give N-[2-(p-hydroxyphenyl)-ethyl]-p-chlorobenzamide.

3. 4-(p-Acetoxybenzylidene)-2-(p-chlorophenyl)-5-oxazolone.

4. 2-(p-Chlorobenzamido)-3-(p-hydroxyphenyl)-acrylic acid, its salts, esters and amides.

5. 1-(p-Chlorobenzamido)-2-(p-hydroxyphenyl)-ethylene, its salts and esters.

## Revendications

1. Procédé pour la préparation de N-[2-(p-hydroxyphényl)éthyl]-p-chlorobenzamide, caractérisé en ce qu'il comprend les stades suivants:

a) on transforme la N-(p-chlorobenzoyl)glycine avec du p-hydroxybenzaldéhyde dans les conditions d'une condensation aldol en une 4-(p-acyloxybenzylidène)-2-(p-chlorophényle)-5-oxazolone,

b) on transforme la 4-(p-acyloxybenzylidène)-2-(p-chlorophényl)-5-oxazolone par saponification basique en acide 2-(p-chlorobenzamido)-3-(p-hydroxyphényl)acrylique,

c) on transforme l'acide 2-(p-chlorobenzamide)-3-(p-hydroxyphényl)acrylique par décarboxylation en 1-(p-chlorobenzamido)-2-(p-hydroxyphényl)éthylène, et

d) on transforme le 1-(p-chlorobenzamido)-2-(p-hydroxyphényl)éthylène par hydrogénation en N-[2-(p-hydroxyphényl)éthyl]-p-chlorobenzamide.

2. Procédé de préparatioon de N-[2-(p-hydroxyphényl)éthyl]-p-chlorobenzamide, caractérisé en ce qu'il comprend les stades suivants:

a) on transforme la N-(p-chlorobenzoyl)-glycine avec du p-hydroxybenzaldéhyde en présence d'acétate de sodium et d'acétanhydride en 4-(p-acétoxybenzylidène)-2-(p-chlorophényle)-5-oxazolone,

b) on transforme la 4-(p-acétoxybenzylidène)-2-(p-chlorophényle)-5-oxazolone par saponification avec de l'hydroxyde de sodium en acide 2-(p-chlorobenzamido)-3-(p-hydroxyphényl)-acrylique,

c) on transforme l'acide 2-(p-chlorobenzamido)-3-(p-hydroxyphényl)acrylique par décarboxylation en présence de cuivre et de quinoléine en 1-(p-chlorobenzamido)-2-(p-hydroxyphényl) éthylène, et

d) on transforme le 1-(p-chlorobenzamido)-2-(p-hydroxyphényl)éthylène par hydrogénation catalytique en N-[2-(p-hydroxyphényl)éthyl]-p-chlorobenzamide.

3. La 4-(p-acétoxybenzylidène)-2-(p-chlorophényle)-5-oxazolone.

4. L'acide 2-(p-chlorobenzamido)-3-(p-hydroxyphényl)acrylique, ses sels, ses esters et ses amides.

5. Le 1-(p-chlorobenzamido)-2-(p-hydroxyphényl)éthylène, ses sels et ses esters.